# EUROPÄISCHE PATENTSCHRIFT

(11) **EP 2 678 157 B1**
(45) Veröffentlichungstag und Bekanntmachung des Hinweises auf die Patenterteilung: **04.07.2018**
(21) Anmeldenummer: 12700610.4
(22) Anmeldetag: 17.01.2012
(51) Int. Cl.: B32B 25/00, E04F 15/10

(54) **BODENBELAG**
FLOOR COVERING
REVÊTEMENT DE SOL

(30) Priorität: 23.02.2011 DE 102011012169
(43) Veröffentlichungstag der Anmeldung: 01.01.2014
(73) Patentinhaber: Nora Systems GmbH, 69469 Weinheim (DE)
(72) Erfinder: KELLER, Uwe, 68794 Oberhausen-Rheinhausen (DE); GRUN, Gregor, 66482 Zweibrücken (DE); BUTSCHER, Alfons, 69488 Birkenau (DE)
(74) Vertreter: Reiser & Partner Patentanwälte mbB
(86) Internationale Anmeldenummer: PCT/EP2012/000176
(87) Internationale Veröffentlichungsnummer: WO 2012/113493

(56) Entgegenhaltungen:
- EP-A1- 1 793 032
- DE-A1-102006 018 099

## Beschreibung

Die Erfindung betrifft einen Bodenbelag aus elastomerem Material und ein Verfahren zu dessen Herstellung.

Bodenbeläge aus elastomerem Materialien werden meist in Fliesenform, beispielsweise als quadratische Platten, mit einer Kantenlänge von 50 cm oder 1 m hergestellt. Aufgrund der Materialeigenschaften des Elastomers weisen derartige Bodenbeläge in ungebundenem, lose verlegtem Zustand deutliche temperaturabhängige Dimensionsänderungen auf. Für die in elastomeren Bodenbelägen häufig verwendeten elastomeren Werkstoffe Styrol, Butadien, Kautschuk und Nitrilbutadienkautschuk liegen die thermischen Ausdehnungskoeffizienten beispielsweise bei etwa 0,02% je Grad Temperaturänderung. Ein derartiger Ausdehnungskoeffizient führt dazu, dass eine Fliese eines elastomeren Bodenbelags mit einer Kantenlänge von 1 Meter bei einer Temperaturänderung von 10° Celsius eine Längenänderung von mehr als 1,5 mm erfährt. Aus diesem Grund ist es erforderlich, elastomere Bodenbeläge beim Verlegen mit dem Untergrund zu verkleben, um eine einheitliche Fläche ohne Fugen oder Stippnähte zu erhalten. Zum Verkleben wird meist ein pastöser Klebstoff verwendet, der vollflächig auf den Untergrund oder die Bodenbelagsfliesen aufgebracht wird.

Jedoch ist das vollflächige Verkleben eines Bodenbelags einerseits aufwändig und erfordert je nach Untergrund möglicherweise den Einsatz eines gesundheitsgefährdenden Klebstoffs. Ferner sind Untergründe denkbar, bei denen ein Verkleben überhaupt gar nicht möglich ist.

Aus der EP 1 793 032 A1 ist ein Material für einen Bodenbelag bekannt, welches mehrlagig ausgebildet ist und mit diversen Füllstoffen versehen sein kann.

Aus der DE 10 2006 018 099 A1 ist ein Bodenbelag bekannt, bei welchem eine Effektschicht zur Erzielung einer optischen Wirkung mit Effektpartikeln, beispielsweise Glimmer, versehen ist.

Der Erfindung liegt die Aufgabe zugrunde, einen Bodenbelag aus elastomerem Material bereitzustellen, welcher unverklebt verlegt werden kann.

Diese Aufgabe wird mit den Merkmalen der Patentansprüche 1 und 5 gelöst. Auf vorteilhafte Ausgestaltungen nehmen jeweils die Unteransprüche Bezug.

Zur Lösung der Aufgabe umfasst der Bodenbelag eine erste Lage aus elastomerem Material und eine zweite Lage aus elastomerem Material, wobei beide Lagen mit Füllstoffen versehen sind. Die Füllstoffe sind in den beiden Lagen derart gewählt, dass die zweite Lage in zumindest einer Richtung bei Temperaturänderung einen größeren Ausdehnungskoeffizienten aufweist als die erste Lage und wobei zwischen der ersten Lage und der zweiten Lage eine weitere Lage angeordnet ist. Durch die flächige Verteilung der Füllstoffe ergibt vorzugsweise in Richtung auf alle Kanten, also in alle Hauptrichtungen des Bodenbelags ein unterschiedlicher Ausdehnungskoeffizient zwischen erster und zweiter Lage. Aufgrund der festen Verbindung der ersten und der zweiten Lage ergibt sich beim Abkühlen des Bodenbelags nach der Vulkanisation in dessen Inneren eine Vorspannung, welche dazu führt, dass die Kanten des Bodenbelags, bzw. der Bodenbelagsfliese wie bei einem Bimetall nach unten, d. h. in Richtung des Untergrunds leicht gebogen werden. Dadurch liegt der Bodenbelag nahezu vollflächig auf dem Untergrund auf.

Die zwischen den beiden Lagen angeordnete weitere Lage ist so ausgebildet, dass diese einen möglichst geringen temperaturabhängigen Ausdehnungskoeffizienten aufweist. Durch die feste Einbindung der weiteren Lage zwischen der ersten Lage und der zweiten Lage ist auch der Bodenbelag insgesamt bei Temperaturänderungen dimensionsstabil.

Der Füllstoff enthält erfindungsgemäß plättchenförmige Partikel. Plättchenförmige Partikel sind dadurch gekennzeichnet, dass sie flach ausgebildet sind und dass deren Höhe kleiner ist als deren Länge und Breite. Eine derartige plättchenförmige Formgebung weisen beispielsweise Chips oder schichtartige Partikel auf. Die plättchenförmigen Partikel richten sich während der Verarbeitung der Lagen, insbesondere während des Kalandrierens oder während des Verfließens des elastomeren Rohmaterials aus, was dazu führt, dass sich die plättchenförmigen Partikel, fest in die Lagen eingebunden, während des Abkühlens der Lage gegenseitig behindern, dabei jedoch nicht ausweichen können und so die Längenabnahme der Lage begrenzen. Diese vorteilhafte Wirkung ergibt sich insbesondere dann, wenn das Verhältnis von Länge zu Höhe (Aspektverhältnis) der plättchenförmigen Partikel mindestens 10:1 beträgt.

Dabei weist die erste Lage eine größere Menge des ersten Bestandteils des Füllstoffs aufweist als die zweite Lage, wobei bezogen auf die jeweilige Lage der Gewichtsanteil des ersten Bestandteils des Füllstoffs der ersten Lage vorzugsweise wenigstens 10% größer ist als der der zweiten Lage. Bei diesem Mengenverhältnis ist sichergestellt, dass sich innerhalb des Bodenbelags eine ausreichend große Vorspannung ausbildet, durch die der Belag ohne Aufwölben der Kanten nahezu vollflächig auf dem Untergrund aufliegt.

In einer bevorzugten Ausgestaltung besteht die weitere Lage aus einem textilen Flächengebilde. Ein derartiges textiles Flächengebilde kann aus einem Vliesstoff, einem Gelege oder einem Gewebe oder einer Mischform aus den zuvor Genannten bestehen. Als Werkstoff für das textile Flächengebilde kommen vorzugsweise Fasern aus polymeren Werkstoffen oder aus Glasfasern in Betracht. Das textile Flächengebilde lässt sich mit einfachen Mitteln, beispielsweise mittels Vulkanisation oder mittels Kleben fest in die Matrix des Bodenbelags einbinden, so dass es die Ausdehnung der ersten Lage und der zweiten Lage begrenzt und so die Dimensionsstabilität des Bodenbelags insgesamt gewährleistet.

Als elastomeres Grundmaterial sind alle Elastomere geeignet, die für die Anwendung als Bodenbelag geeignet sind. Vorzugsweise umfasst das Grundmaterial die Elastomere Styrol-Butadien-Kautschuk (SBR), Nitril-Butadien-Kautschuk (NBR), Ethylen-Propylen-Kautschuk (EPM), Ethylen-Propylen-Dien-Kautschuk (EPDM), Ethylenvinylacetat (EVA), Chlorsulfonyl-Polyethylen-Kautschuk (CSM), Silikon-Kautschuk (VSI) und/oder Ethylen-Acrylat-Kautschuk (AEM), sowohl schwefelvernetzt, peroxidvernetzt als auch additionsvernetzt. Es können auch Mischungen der genannten Elastomere zur Anwendung gelangen.

Vorzugsweise umfasst die weitere Schicht einen Vliesstoff. Als Material für den Vliesstoff kommt insbesondere Polyester in Frage. Vliesstoffe sind einerseits kostengünstig verfügbar und können andererseits so ausgebildet sein, dass der Ausdehnungskoeffizient des Vliesstoffes richtungsungebunden ist, was bedeutet, dass der Vliesstoff über die Fläche betrachtet in jeder Richtung einen identischen Ausdehnungskoeffizienten aufweist. Dadurch weist auch der Bodenbelag in den die weitere Schicht eingebunden ist entsprechend in alle Richtungen einen identischen Ausdehnungskoeffizienten auf. In diesem Zusammenhang ist insbesondere ein thermisch gebundener Spinnvlies vorteilhaft, weil dieser einen besonders gleichmäßigen Ausdehnungskoeffizienten aufweist, besonders stabil ist und zur Verarbeitung in elastomerverarbeitenden Maschinen geeignet ist.

Die plättchenförmigen Partikel können ein Schichtsilikat, insbesondere Kaolin und/oder Glimmer umfassen. Schichtsilikate weisen einen schichtweisen Aufbau auf, der die Herstellung von besonders flachen, also mit einem großen Unterschied von Länge zu Höhe (Aspektverhältnis), plättchenförmigen Füllstoffen ermöglicht. Darüber hinaus sind insbesondere Kaolin und Glimmer in einer Vielzahl von industriellen Anwendungen bereits bekannt.

Bei dem erfindungsgemäßen Verfahren zur Herstellung eines Bodenbelags werden eine erste Lage mit einer ersten Menge an Füllstoffen hergestellt, eine zweite Lage mit einer zweiten Menge an Füllstoffen hergestellt, eine weitere Lage zwischen der ersten Lage und der zweiten Lage angeordnet und die Lagen stoffschlüssig miteinander verbunden. Erfindungsgemäß werden zwei Lagen mit voneinander unterschiedlichen Mengen an Füllstoffen bereitgestellt, welche dadurch einen unterschiedlichen Ausdehnungskoeffizienten aufweisen. Durch die zwischen den beiden Lagen angeordnete weitere Schicht, vorzugsweise ein textiles Flächengebilde, wird die temperaturabhängige Ausdehnung des Bodenbelags insgesamt begrenzt.

Zum Ausbilden der elastomeren Eigenschaften können die Lagen einem Vulkanisationsprozess unterzogen werden. In diesen Zusammenhang ist vorteilhaft, die Lagen einem gemeinsamen Vulkanisationsprozess zu unterziehen, um die Lagen darüber hinaus untereinander stoffschlüssig zu verbinden. Der Vulkanisationsprozess ist eine kombinierte Wärme- und Druckbehandlung des den Bodenbelag bildenden Rohmaterials, wodurch die erste Lage und die zweite Lage einerseits die gummielastischen Eigenschaften erhalten
und andererseits die Lagen an den einander berührenden Flächen vom Material der jeweils anderen Lage durchdrungen werden, wodurch sich ein besonders fester stoffschlüssiger Verbund ergibt. Vor diesem Hintergrund ist insbesondere die Ausbildung der weiteren Lage aus einem Vliesstoff besonders vorteilhaft. Aufgrund der nicht geschlossenen Matrix des Vliesstoffs, dringt das Material der ersten Lage und der zweiten Lage bei dem Vulkanisationsprozess in die Matrix des Vliesstoffs ein und es ergibt sich ein besonders fester stoffschlüssiger Verbund. Insofern ist die weitere Schicht bei diesem Verfahren an die Lagen anvulkanisiert.

Es ist jedoch auch denkbar, die weitere Schicht mittels Kleben an die erste Lage und die zweite Lage stoffschlüssig anzubinden. Dies ist insbesondere dann vorteilhaft, wenn die erste und/oder die zweite Lage keinen Vulkanisationsprozess benötigen oder dieser bereits abgeschlossen ist.

Der erfindungsgemäß ausgestaltete Bodenbelag kann ohne jedes Bindemittel lediglich auf den Untergrund aufgelegt werden. Insbesondere bei einer Ausbildung des Bodenbelags in Form von Fliesen ist auch nicht erforderlich, die einzelnen Fliesen des Bodenbelags untereinander zu verbinden. Die Fliesen des Bodenbelags werden nebeneinander auf dem Untergrund aufgelegt und bilden einen formschlüssigen Verbund, wobei durch die Vorspannung innerhalb des Materials des Bodenbelags gewährleistet ist, dass sich die Kanten der einzelnen Fliesen des Bodenbelags nicht aufwölben. Ferner ist aufgrund der weiteren Lage gewährleistet, dass der Ausdehnungskoeffizient der Fliesen so begrenzt ist, dass sich auch keine Spalten zwischen den Fliesen bilden.

In einem besonders bevorzugten Verlegeverfahren werden zunächst Fliesen eines Bodenbelags bereitgestellt. Anschließend werden die Fliesen auf der dem Untergrund zugewandten Seite mit einem doppelseitigen Klebeband versehen und auf den Untergrund aufgelegt. Dabei ist vorzugsweise vorgesehen, dass lediglich die Randbereiche der einzelnen Fliesen oder auch nur die den Ecken zugeordneten Bereiche der einzelnen Fliesen mit dem Klebeband versehen werden. Das doppelseitige Klebeband ist vorzugsweise so ausgebildet, dass es in Richtung des Bodenbelags eine starke Klebewirkung entfaltet und in Richtung des Untergrundes nur eine schwache Klebewirkung entfaltet. Demnach ist die Klebewirkung des doppelseitigen Klebebandes gegenüber dem Untergrund schwächer als gegenüber den Fliesen des Bodenbelags. Dadurch ist es möglich, den Bodenbelag jederzeit einfach wieder zu entnehmen. In einer weiteren vorteilhaften Ausgestaltung werden an Stelle eines Klebebands Klebeetiketten in die Eckbereiche der Fliesen aufgeklebt, wobei die schwache Klebewirkung der Etiketten zum Untergrund ausgerichtet ist. Dieses weist den Vorteil auf, dass es mehrfach wiederverwendbar ist, wodurch die so ausgerüsteten Bodenbelagsfliesen insbesondere für den Messebau oder auf Doppelböden geeignet sind.

Ein Ausführungsbeispiele des erfindungsgemäßen Bodenbelags wird nachfolgend anhand der Figur näher erläutert. Diese zeigt schematisch:

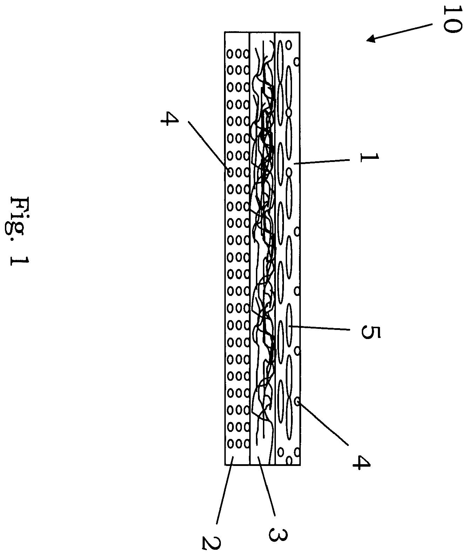

- Fig. 1: einen mehrlagigen Bodenbelag.

Figur 1 zeigt einen mehrlagigen elastomeren Bodenbelag 10. Eine erste Lage 1 besteht aus elastomerem Material auf der Basis von Styrol-Butadien-Kautschuk (SBR) und ist mit Füllstoffen 4 versehen. Die Füllstoffe 4 enthalten plättchenförmige Partikel 5, von denen wenigstens ein Teil aus Glimmer gebildet ist. Dabei sind die plättchenförmigen Partikel 5 derart ausgebildet, dass das Verhältnis von Länge zu Höhe (Aspektverhältnis) mindestens 10:1 beträgt. In einer weiteren Ausgestaltung besteht der erste Bestandteil aus ebenfalls schichtförmigem Kaolin oder aus einer Kombination aus Kaolin und Glimmer. Die zweite Lage 2 basiert ebenfalls auf Styrol-Butadien-Kautschuk (SBR) und ist ebenfalls mit Füllstoffen 4 versehen, jedoch ist in der zweiten Lage 2 keine signifikante Menge an plättchenförmigen Partikeln 5 vorgesehen. Durch diese erfindungsgemäße Wahl des Füllstoffs 4 weist die zweite Lage 2 bei Temperaturänderung einen größeren Ausdehnungskoeffizienten auf als die erste Lage 1, so dass sich innerhalb des Bodenbelags 10 eine Vorspannung ergibt, die dazu führt, dass der sich, insbesondere bei der Ausgestaltung des Bodenbelags 10 als Fliese, der Bodenbelag wie bei einem Bimetall in Richtung auf die Kanten krümmt. Insgesamt ergibt sich so ein gewölbter Bodenbelag 10, dessen Kanten sich in unbelastetem Zustand selbständig in Richtung des Untergrundes biegen. Ist der Bodenbelag 10 bestimmungsgemäß auf einen Untergrund aufgelegt, erstreckt sich der Bodenbelag 10 entlang des Untergrundes, wobei aus der behinderten Biegung im Inneren des Bodenbelags 10 Spannungen resultieren, die ein planes Aufliegen des Bodenbelags 10 auf dem Untergrund gewährleisten. Es ist auch denkbar, dass auch die zweite Lage 2 plättchenförmige Partikel gemäß dem ersten Bestandteil 5 aufweist. Jedoch ist bezogen auf die jeweilige Lage der Gewichtsanteil der plättchenförmigen Partikel 5 erfindungsgemäß in der zweiten Lage 2 stets geringer als in der ersten Lage 1, wobei der Gewichtsunterschied wenigstens 10% betragen sollte.

Zwischen der ersten Lage 1 und der zweiten Lage 2 ist eine weitere Lage 3 angeordnet, die aus einem thermisch gebundenen Polyester-Spinnvlies gebildet ist. Ein bevorzugtes Flächengewicht des Polyester-Spinnvlieses beträgt zwischen 100 und 200 g/m². Die weitere Lage 3 weist eine hohe Zugfestigkeit sowohl in Längs- als auch in Querrichtung auf. Ferner weist die weitere Lage einen besonders geringen Ausdehnungskoeffizienten auf, so dass auch der Bodenbelag 10 insgesamt aufgrund der festen Einbindung der weiteren Lage 3 in die Matrix des Bodenbelags 10 nur noch einen geringen Ausdehnungskoeffizienten aufweist.

Ein beispielhafter erfindungsgemäßer Bodenbelag weist folgende Ausgestaltung auf:
Die erste Lage 1 enthält SBR, diverse Füllstoffe 4, wobei der Füllstoff 4 plättchenförmige Partikel 5 gebildet aus Glimmer enthält. Ferner enthalten die Füllstoffe 4 ein Vulkanisationsmittel. Die zweite Lage 2 enthält ebenfalls SBR und diverse Füllstoffe 4, darin enthalten ist auch ein Vulkanisationsmittel, jedoch enthalten die für die zweite Lage 2 vorgesehenen Füllstoffe 4 keine signifikante Menge plättchenförmiger Partikel 5. Die weitere Lage 3 ist aus einem thermisch gebundenen Polyester-Spinnvlies mit einem Flächengewicht von 130 g/m² gebildet.

Zur Herstellung des Bodenbelags 10 wird eine erste Lage 1 mit einer ersten Menge an Füllstoffen 4 hergestellt. Dabei ist der Füllstoff 4, der für die erste Lage 1 vorgesehen ist, mit einer bestimmten Menge plättchenförmiger Partikel 5 versehen. Ferner wird eine zweite Lage 2 mit einer zweiten Menge an Füllstoffen 4 hergestellt, wobei der für die zweite Lage 2 vorgesehene Füllstoff 4 keinen oder wenigstens 10% weniger plättchenförmige Partikel 5 aufweist. Eine weitere Lage 3 wird bereitgestellt und zwischen der ersten Lage 1 und der zweiten Lage 2 angeordnet. Die weitere Lage 3 besteht aus einem thermisch gebundenen Spinnvlies. Schließlich werden die Lagen 1, 2, 3 stoffschlüssig miteinander verbunden. Vorzugsweise erfolgt das Verbinden mittels eines Vulkanisationsprozesses bei dem die sandwichartig übereinander angeordneten Lagen 1, 2, 3 einer kombinierten Wärme- und Druckbehandlung unterzogen werden.

Dadurch bilden sich die gummielastischen Eigenschaften der ersten Lage 1 und der zweiten Lage 2 heraus und das Material der ersten Lage 1 und der zweiten Lage 2 dringt teilweise in die Poren der weiteren Lage 3 ein, wodurch sich ein fester Verbund herausbildet. Dadurch ist die weitere Lage 3 an die erste Lage 1 und an die zweite Lage 2 anvulkanisiert. In einem weiteren vorteilhaften Verfahren wird die weitere Lage 3 mit der ersten Lage 1 und der zweiten Lage 2 mittels einer Klebeschicht stoffschlüssig verbunden. Dies erfolgt mittels eines Klebstoffs, der vollflächig jeweils auf wenigstens einer der einander berührenden Seiten der Lagen 1, 2, 3 aufgebracht wird. Auch hier kann der Bodenbelag 10 zusätzlich einer Wärme- und/oder Druckbehandlung unterzogen werden.

Bei einem erfindungsgemäßen Verlegeverfahren werden zunächst Fliesen eines Bodenbelags bereitgestellt. Anschließend werden die Fliesen auf der dem Untergrund zugewandten Seite in den Bereichen der Kanten und/oder der Ecken mit einem doppelseitigen Klebeband oder mit Klebeetiketten versehen und anschließend nebeneinander auf den Untergrund aufgelegt. Das doppelseitige Klebeband ist dabei so ausgebildet, dass es in Richtung des Bodenbelags eine stärkere Klebewirkung entfaltet als in Richtung des Untergrundes.

## Patentansprüche

1. Bodenbelag (10), umfassend eine erste Lage (1) aus elastomerem Material und eine zweite Lage (2) aus elastomerem Material, wobei beide Lagen (1, 2) mit Füllstoffen (4) versehen sind, wobei der Füllstoff (4) plättchenförmige Partikel (5) enthält, wobei die Füllstoffe (4) in den beiden Lagen (1, 2) jeweils derart gewählt sind, dass die zweite Lage (2) zumindest in einer Richtung bei Temperaturänderung einen größeren Ausdehnungskoeffizienten aufweist als die erste Lage (1), wobei sich die Menge an Füllstoffen (4) in der ersten Lage (1) von der Menge an Füllstoffen in der zweiten Lage (2) unterscheidet, wobei die erste Lage (1) eine größere Menge der plättchenförmigen Partikel (5) aufweist als die zweite Lage (2), wobei bezogen auf die jeweilige Lage der Gewichtsanteil der plättchenförmigen Partikel (5) der ersten Lage (1) wenigstens 10% größer ist als der der zweiten Lage (2) und wobei zwischen der ersten Lage (1) und der zweiten Lage (2) eine weitere Lage (3) angeordnet ist.

2. Bodenbelag nach Anspruch 1, **dadurch gekennzeichnet, dass** die weitere Lage (3) einen Vliesstoff umfasst.

3. Bodenbelag nach Anspruch 1, **dadurch gekennzeichnet, dass** die plättchenförmigen Partikel (5) ein Verhältnis von Länge zu Höhe von mindestens 10:1 aufweisen.

4. Bodenbelag nach Anspruch 1 oder 3, **dadurch gekennzeichnet, dass** die plättchenförmigen Partikel (5) Kaolin und/oder Glimmer umfassen.

5. Verfahren zur Herstellung eines elastomeren Bodenbelags nach einem der voranstehenden Ansprüche bei dem
• eine erste Lage (1) mit einer ersten Menge an Füllstoffen (4) enthaltend plättchenförmige Partikel (5) hergestellt wird;
• eine zweite Lage (2) mit einer zweiten Menge an Füllstoffen (4) enthaltend plättchenförmige Partikel (5) hergestellt wird, wobei die erste Lage (1) eine größere Menge der plättchenförmigen Partikel (5) aufweist als die zweite Lage (2), wobei bezogen auf die jeweilige Lage der Gewichtsanteil der plättchenförmigen Partikel (5) der ersten Lage (1) wenigstens 10% größer ist als der der zweiten Lage (2);
• eine weitere Lage (3) zwischen der ersten Lage (1) und der zweiten Lage (2) angeordnet wird und
• die Lagen (1, 2, 3) stoffschlüssig miteinander verbunden werden.

6. Verfahren nach Anspruch 5, **dadurch gekennzeichnet, dass** die Lagen (1, 2) einem Vulkanisationsprozess unterzogen werden.

7. Verfahren nach Anspruch 5 oder 6, **dadurch gekennzeichnet, dass** die weitere Lage (3) an die erste Lage (1) und die zweite Lage (2) anvulkanisiert ist.

8. Verfahren nach Anspruch 5, **dadurch gekennzeichnet, dass** die weitere Lage (3) mit der ersten Lage (1) und der zweiten Lage (2) mittels einer Klebeschicht stoffschlüssig verbunden ist.

## Claims

1. Floor covering (10), comprising a first ply (1) of elastomeric material and a second ply (2) of elastomeric material, both plies (1, 2) being provided with fillers (4), said fillers (4) containing platelet-shaped particles (5), wherein the fillers (4) in the two plies (1, 2) are chosen such that in the event of a change in temperature the second ply (2) exhibits a greater coefficient of expansion than the first ply (1) in at least one direction, wherein the quantity of fillers (4) in the first ply (1) differs from the quantity of fillers in the second ply (2), wherein the first ply (1) exhibits a greater quantity of the platelet-shaped particles (5) than the second ply (2), wherein in relation to the respective ply the proportion by weight of the platelet-shaped particles (5) of the first ply (1) is at least 10 % greater than that of the second ply (2) and wherein an additional ply (3) is arranged between the first ply (1) and the second ply (2).

2. Floor covering according to claim 1, **characterised in that** the additional ply (3) comprises a nonwoven fabric.

3. Floor covering according to claim 1, **characterised in that** the platelet-shaped particles (5) exhibit a ratio of length to height of at least 10:1.

4. Floor covering according to claim 1 or 3, **characterised in that** the platelet-shaped particles (5) comprise kaolin and/or mica.

5. Method of manufacturing an elastomeric floor covering in accordance with any one of the preceding claims, wherein
• a first ply (1) is manufactured with a first quantity of fillers (4) containing platelet-shaped particles (5);
• a second ply (2) is manufactured with a second quantity of fillers (4) containing platelet-shaped particles (5), wherein the first ply (1) exhibits a greater quantity of the platelet-shaped particles (5) than the second ply (2), wherein in relation to the respective ply the proportion by weight of the platelet-shaped particles (5) of the first ply (1) is at least 10 % greater than that of the second ply (2) ;
• an additional ply (3) is arranged between the first ply (1) and the second ply (2) and
• the plies (1, 2, 3) are interconnected by means of a substance bond.

6. Method according to claim 5, **characterised in that** the plies (1, 2) are subjected to a vulcanisation process.

7. Method according to claim 5 or 6, **characterised in that** the additional ply (3) is vulcanised on the first ply (1) and the second ply (2).

8. Method according to claim 5, **characterised in that** the additional ply (3) is substance-bonded to the first ply (1) and the second ply (2) by means of an adhesive layer.

## Revendications

1. Revêtement de sol (10), comprenant une première couche (1) en matière élastomère et une deuxième couche (2) en matière élastomère, les deux couches (1, 2) étant munies de matières de remplissage (4), la matière de remplissage (4) contenant des particules (5) en forme de plaquettes, les matières de remplissage (4) dans les deux couches (1, 2) étant respectivement sélectionnées de manière à ce que la deuxième couche (2) présente, au moins dans une direction, un coefficient de dilatation plus élevé, en cas de variation de température, que la première couche (1), la quantité de matières de remplissage (4) dans la première couche (1) se distinguant de la quantité de matières de remplissage dans la deuxième couche (2), la première couche (1) présentant une plus grande quantité de particules (5) en forme de plaquettes que la deuxième couche (2), par rapport à la couche respective, la fraction pondérale des particules (5) en forme de plaquettes de la première couche (1) étant au moins 10 % supérieure à celle de la deuxième couche (2), et une couche supplémentaire (3) étant disposée entre la première couche (1) et la deuxième couche (2).

2. Revêtement de sol selon la revendication 1, **caractérisé en ce que** la couche supplémentaire (3) comprend un non-tissé.

3. Revêtement de sol selon la revendication 1, **caractérisé en ce que** les particules (5) en forme de plaquettes présentent un rapport longueur/hauteur d'au moins de 10 : 1.

4. Revêtement de sol selon la revendication 1 ou 3, **caractérisé en ce que** les particules (5) en forme de plaquettes comprennent du kaolin et/ou du mica.

5. Procédé de fabrication d'un revêtement de sol élastomère selon l'une des revendications précédentes, dans lequel
• une première couche (1) comprenant des particules (5) en forme de plaquettes contenant une première quantité de matières de remplissage (4) est fabriquée ;
• une deuxième couche (2) comprenant des particules (5) en forme de plaquettes contenant une deuxième quantité de matières de remplissage (4) est fabriquée, la première couche (1) présentant une quantité plus élevée de particules (5) en forme de plaquettes que la deuxième couche (2), par rapport à la couche respective, la fraction pondérale des particules (5) en forme de plaquettes de la première couche (1) étant au moins 10 % supérieure à celle de la deuxième couche (2) ;
• une couche supplémentaire (3) est disposée entre la première couche (1) et la deuxième couche (2), et
• les couches (1, 2, 3) sont reliées entre elles par adhérence de matière.

6. Procédé selon la revendication 5, **caractérisé en ce que** les couches (1, 2) sont soumises à un processus de vulcanisation.

7. Procédé selon la revendication 5 ou 6, **caractérisé en ce que** la couche supplémentaire (3) est vulcanisée sur la première couche (1) et sur la deuxième couche (2).

8. Procédé selon la revendication 5, **caractérisé en ce que** la couche supplémentaire (3) est reliée à la première couche (1) et à la deuxième couche (2) par adhérence de matière au moyen d'une couche de colle.
